Europäisches Patentamt

(19) **European Patent Office**

**Office européen des brevets**

(11) Publication number: **0 214 174**

**B1**

## (12) EUROPEAN PATENT SPECIFICATION

(45) Date of publication of the patent specification:
**19.04.89**

(51) Int. Cl.⁴: **F 16 C 7/02**

(21) Application number: **86901111.4**

(22) Date of filling: **10.02.86**

(86) International application number:
**PCT/GB 86/00071**

(87) International publication number:
**WO 86/04650 (14.08.86 Gazette 86/18)**

(54) **FIBRE REINFORCED PLASTIC CONNECTING ROD.**

(30) Priority: **12.02.85 GB 8503535**

(43) Date of publication of application:
**18.03.87 Bulletin 87/12**

(45) Publication of the grant of the patent:
**19.04.89 Bulletin 89/16**

(84) Designated Contracting States:
**AT BE CH DE FR GB IT LI LU NL SE**

(56) References cited:
**EP-A-0 033 765**
**DE-A-3 207 573**
**DE-A-3 329 001**
**GB-A-1 364 317**

**Patents Abstracts of Japan, vol. 7, no. 279, (M-262)(1424), 13. December 1983**
**Patents Abstracts of Japan, vol. 9, no. 128, (M-384)(1851), 4. June 1985**

(73) Proprietor: **The Secretary of State for Trade and Industry in Her Britannic Majesty's Government of the United Kingdom of Great Britain and, Northern Ireland 1 Victoria Street, London SW1H OET (GB)**

(72) Inventor: **HUGHES, Joseph, David, Aled, Hecklebirnie, Sandilands Lanarkshire ML11 9TR (GB)**
Inventor: **WOOTON, Alan, James, 10 Roland Crescent, Newton Mearne Glasgow G77 5JT (GB)**
Inventor: **LEE, Walter, Alick, 5 Newton Road, Strathaven ML10 6BQ (GB)**
Inventor: **MITCHELL, Alexander, Marshall, 101 Ballochmyle, East Kilbride Glasgow G74 3RT (GB)**

(74) Representative: **Beckham, Robert William, Procurement Executive Ministry of Defence Patents 1A(4), Room 2014, Empress State Building Lillie Road London SW6 1TR (GB)**

LIBER, STOCKHOLM 1989

## Description

The present invention relates to fibre reinforced plastic connecting rods for machinery in which reciprocating motion is converted to rotary motion or vice versa.

There are many industrial uses of machines in which reciprocating motion is converted into rotary motion, the best known example being the internal combustion engine. In this type of machine a significant proportion of the energy lost results from the need to overcome the inertia of connecting rods. Connecting rods are used to transfer the reciprocating motion of pistons to rotary motion of a crankshaft. Connecting rods themselves have a motion which is a combination of linear and rotary.

Conventionally connecting rods have been made of metal, and in order to cut down the energy loss considerable effort has been devoted into reducing their weight. Efforts to reduce weight are, of course, complicated by the fact that connecting rods not only have to carry large forces to fulfil their function but have to sustain the effects of inertia forces due to their own weight. Recent advances in the technology of Fibre Reinforced Plastics (FRP) materials have made these materials suitable for use in the manufacture of connecting rods.

Connecting rods conventionally consist of a rod member having at one end, usually referred to as the little end, means for attaching the rod to a piston, and at the other end, commonly referred to as the big end, means for attaching the connecting rod to a crankshaft. To enable the rod to be connected to a crankshaft it is usually formed in two parts, having a cap member attachable to the rod member, the end of the rod and cap each including semi-circular portions which form a complete circle when the two are joined together.

The usual method of joining the rod and cap involves the use of nuts on bolts which pass through bores in the rod end and in the cap. This method is not ideal for connecting rods of FRP material. Drilling of bore holes in FRP material results in undesireable fracture of fibres. Manufacturing techniques for fabricating ends with bores therein in which fibres are not fractured are complicated and expensive. Also in FRP connecting rods with this type of end cap joint the cross-sectional area of the rod in the region of the bores has to be increased in order to withstand operational stresses. The increase can be such as to make such FRP connecting rods unsuitable for use in existing engine designs.

Patent Application DE-A-3 329 001 describes a connecting rod in which an FRP stiffening member is positioned between little and big end bearing shells, the little end bearing shell being encompassed in a circular first metal insert and the big end bearing shell being separated from the stiffening member by a second metal insert, illustrated as of semi-circular form. Two tie members adjacent the big end are held in position by an endless loop of fibres which pass round each tie member and around the first metal insert. A cap member is secured to the connecting rod by studs or bolts integral with or passing through the tie members.

The use of several separate components including an endless loop of fibres results in this arrangement being complicated and relatively expensive.

The object of the invention is to provide an improved design of FRP connecting rod that overcomes the above-mentioned problems.

According to the present invention a connecting rod having a little end and a big end and including a Fibre Reinforced Plastic rod member, a cap member, and means including a metal member whereby the cap member can be secured to the rod member is characterised in that the rod member includes a locally expanded end which is held captive in a re-entrant recess in the metal member to rigidly attach the rod member to the metal member.

According to one form of the invention a rod member of rectangular section is locally expanded on two opposite sides in wedge-like fashion and dove-tailed into a wedge-shaped channel in the metal member. Alternatively the end of the rod might be caused to fit into a frusto-pyramidal or frusto-conical cut-out in the metal member by, for example, injection moulding of thermoplastic material under appropriate conditions of temperature and pressure.

The cap member may be formed from metal, from FRP material, or from a combination of metal and FRP material.

Some embodiments of the invention will now be described, by way of example only, with reference to the accompanying diagrammatic drawings, of which:

Figure 1 is an elevation, partly in section, of one embodiment of the invention,

Figure 2 is an elevation of a modified form of the embodiment of Figure 1, and

Figure 3 is an elevation in section along line X - X of Figure 2.

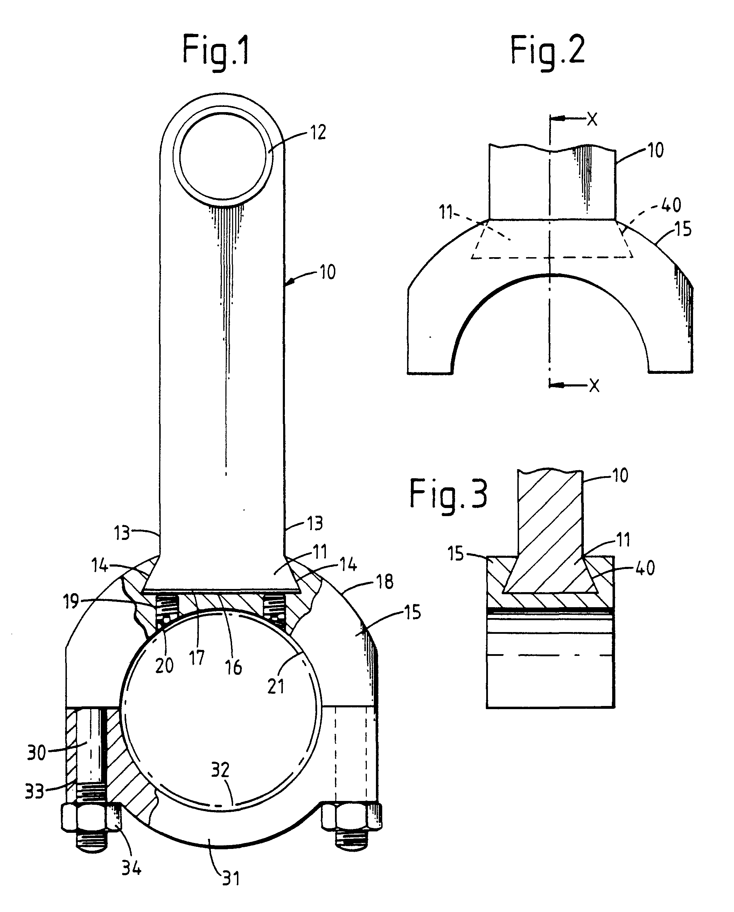

An embodiment of the invention (Figure 1) has a FRP material rod member 10 of substantially rectangular form. An end 11 remote from a little end 12 of the rod member has two opposite faces 13 locally expanded to form wedge shaped projections 14. A metal member 15 of substantially semi-annular or saddle form has a wedge shaped re-entrant recess 16 extending therethrough normal to the plane of the member 15 and extending inwardly from a midpoint of an outer surface 18 thereof. Studs 30 are secured to ends of the member 15 and extend parallel to one another and to an axis of the rod 10.

The end 11 of the rod 10 is introduced into the recess 16 to form a dove tail joint. With this embodiment of the invention it may sometimes be advantageous, in order to ensure a tight fit between the wedge shaped projections 14 and sides of the recess 16, to fit a shim 17 between a base of the recess 16 and the end 11 of the rod 10. Complete rigidity can be ensured by the use of grub screws such as those shown at 19 which are screwed into tapped holes 20 which enter inwardly from an inner surface 21 of member 15. The screws 19 are preferably of a length such that when tight they stand proud of the surface 21. They are then machined flush with the surface 21. When the connecting rod is assembled the screws 19 are locked in position by the presence of a bearing shell (not shown with member 15).

A cap member 31 (shown with a bearing shell 32) is secured to the member by the studs 30, which pass through bores 33, and nuts 34.

In an alternative form of the last above described embodiment (Figures 2 and 3) the member 15 has a frusto-pyramidal recess 40 therein. An end 11 of a rod member 10 is expanded into the recess 40 to be secured therein. Expansion of the end 11 can be accomplished by, for example, injection moulding a short-fibre reinforced thermoplastic material around the end 11 and into the recess 40. The end 11 might have means, such as an annular channel (not shown) to which the thermoplastic material becomes keyed.

The recess 40 can be formed by machining, or the member 15 can be formed in two annularly divided parts, with slots therein. The two parts can then be joined, such that the slots combine to give the recess 40 by, for example, welding or bolting.

When the member 15 is formed in two parts they may be assembled together round an end 11 of a rod 10 which has been pre-formed to the shape of the recess 40. In this case it might be necessary, for complete rigidity, to use shims such as 17, grub screws such as 19 and tapped holes such as 20 similar to those in the embodiment described above with reference to Figure 1.

It might also be possible, depending on the nature of the FRP material used for rod member 10, to cast metal members 15 round a preformed end 11 of the rod member 10.

The rigidly attached rod member 10 and metal member 15 can be connected to a cap member 31 by means other than those described above with reference to Figure 1. For example holes (not shown) in the member 15 may be tapped to allow bolts to be screwed therein after passage through holes 33 in the cap member 15.

Those skilled in the art will realise that many alternative embodiments of the invention are possible. FRP materials suitable for use with the invention will be well known to those skilled in the art and hence will not be described in detail herein.

Various constructions of rod members 10 will be readily apparent to those skilled in the art.

When a rod member 40 of circular cross-section can advantageously be used it may be secured to a metal member 44 by means similar to those of the embodiments described above with reference to Figures 2 and 3 but using a frusto-conical recess 40. Other shapes of recess suitable for use with the invention - for example recesses having curved faces - will be readily apparent.

**Claims**

1. A connecting rod having a little end (12) and a big end and including a Fibre Reinforced Plastic rod member (10), a cap member (31) and a metal member (15) whereby the cap member (31) is secured to the rod member (10), characterised in that the rod member (10) includes a locally expanded end (14) which is held captive in a re-entrant recess (16) in the metal member (15) to rigidly attach the rod member (10) to the metal member (15).

2. A connecting rod as claimed in Claim 1 characterised in that the metal member (15) is in the form of a saddle.

3. A connecting rod as claimed in Claim 1 or in Claim 2 characterised in that the end (11) of the rod member (10) is of rectangular cross-section and has opposite sides expanded to form wedge shaped projections (14) which bear against sides of a re-entrant recess (16) in the metal member (15).

4. A connecting rod as claimed in Claim 3 characterised in having means (17, 19) for forcing the wedge shaped projections (14) into pressurised contact with the sides of the recess (16).

5. A connecting rod as claimed in Claim 4 characterised in that the means for forcing the wedge shaped projections (14) into contact with the sides of the recess (16) include a shim (17) and screws (19) in bores (20) in the metal member (15).

6. A connecting rod as claimed in Claim 2 characterised in that the end of the rod member (10) is of rectangular cross-section and the recess (16) is of frusto-pyramidal form.

7. A connecting rod as claimed in Claim 2 characterised in that the end of the rod member (10) is of circular cross-section and the recess (16) is of frusto-conical form.

8. A connecting rod as claimed in any one of Claims 3, 6 or 7 characterised in that the metal member (15) is formed in two parts, each part having half of the recess (16) therein, the two parts being secured rigidly together.

9. A connecting rod as claimed in any one of Claims 3 to 8 characterised in that the end of the rod member (10) is secured in the recess (16) by the expansion thereof by injection moulding therein of material.

10. A connecting rod as claimed in Claim 9 characterised in that the material is a thermoplastic reinforced with short fibres.

11. A connecting rod as claimed in Claim 9 or Claim 10 wherein the end (11) of the rod member (10) is adapted such that the injection moulded material becomes keyed thereon.

12. A connecting rod as claimed in Claim 8 characterised in that the two parts are positioned around the end (11) of the rod member (10) before being secured together.

13. A connecting rod as claimed in Claim 12 characterised in having means for forcing the end of the rod member (10) into pressurised contact with sides of the recess (16).

14. A connecting rod as claimed in Claim 13 characterised in that the means for forcing the end of the rod member (10) into pressurised contact with sides of the recess (16) include a shim (17) and screws (19) in bores (20) of the metal member (15).

15. A connecting rod as claimed in any one of Claims 1, 2, 6 or 7, characterised in that the metal member (15) is cast into position around the end (11, 14) of the rod member (10).

16. A connecting rod as claimed in any one of Claims 1 to 15 characterised in that means for securing the rod member (10) to the cap member (31) also includes studs (30) which are secured to the metal member (15) and which pass through holes (33) in the cap member (31).

17. A connecting rod as claimed in any one of Claims 1 to 15 characterised in that the means for securing the rod member (10) to the cap member (31) also includes bolts which pass through holes in the metal member (15) and in the cap member (31).

18. A connecting rod as claimed in any one of Claims 1 to 15 characterised in that the means for securing the rod member (10) to the cap member (31) includes bolts which pass through holes in the cap member (31) and tapped holes in the metal member (15).

18. A machine in which reciprocating motion is converted into rotary motion, or vice versa, including a connecting rod as claimed in any one of Claims 1 to 18.

**Patentansprüche**

1. Pleuelstange mit einem Gelenkkopf (12) und einem Lagerkopf, einem faserverstärkten Kunststoffschaft (10), einem Lagerbügel (31) und einem den Lagerbügel (31) mit dem Schaft (10) verbindenden metallenen Pleuelkopf (15),

dadurch gekennzeichnet, daß der Schaft (10) ein erweitertes Schaftende (11, 14) aufweist, das in einer mit einspringenden Wandungen versehenen Ausnehmung (16) des metallenen Pleuelkopfes (15) eingespannt ist und so den Schaft (10) starr mit diesem verbindet.

2. Pleuelstange nach Anspruch 1, dadurch gekennzeichnet, daß der metallene Pleuelkopf (15) sattelförmig ausgebildet ist.

3. Pleuelstange nach Anspruch 1 oder 2, dadurch gekennzeichnet, daß das erweiterte Schaftende (11, 14) rechteckigen Querschnitt hat und sich mit mindestens zwei einander gegenüberliegenden Wandflächen (14) keilartig erweitert, die an entsprechenden Wandflächen der Ausnehmung (16) des metallenen Pleuelkopfes (15) anliegen.

4. Pleuelstange nach Anspruch 3, dadurch gekennzeichnet, daß dieselbe Mittel (17, 19) aufweist, mittels welcher die keilartig erweiterten Wandflächen (14) des Schaftendes (11, 14) an die entsprechenden Wandflächen der Ausnehmung (16) des metallenen Pleuelkopfes (15) angepreßt sind.

5. Pleuelstange nach Anspruch 4, dadurch gekennzeichnet, daß die Mittel zur Anpressung der keilartig erweiterten Wandflächen (14) des Schaftendes (11, 14) an die entsprechenden Wandflächen der Ausnehmung (16) des metallenen Pleuelkopfes (15) mindestens ein Beilageblech (17) und/oder in Gewindelöcher des metallenen Pleuelkopfes (15) eingeschraubte Schrauben (19) umfassen.

6. Pleuelstange nach Anspruch 2, dadurch gekennzeichnet, daß das erweiterte Schaftende (11) rechteckigen Querschnitt hat und daß dieses Schaftende (11) und die Ausnehmung (16) des metallenen Pleuelkopfes (15) jeweils pyramidenstumpfförmig ausgebildet sind.

7. Pleuelstange nach Anspruch 2, dadurch gekennzeichnet, daß das erweiterte Schaftende (11) kreisförmigen Querschnitt hat und daß dieses Schaftende und die Ausnehmung des metallenen Pleuelkopfes (15) jeweils kegelstumpfförmig ausgebildet sind.

8. Pleuelstange nach einem der Ansprüche 3, 6 oder 7, dadurch gekennzeichnet, daß der metallene Pleuelkopf (15) zweiteilig ausgebildet ist und daß jeder Teil desselben eine halbe Ausnehmung (16) aufweist und daß die beiden Pleuelkopfteile starr zusammengespannt sind.

9. Pleuelstange nach einem der Ansprüche 3 bis 8, dadurch gekennzeichnet, daß das erweiterte Schaftende (11) in der Ausnehmung (16) des metallenen Pleuelkopfes (15) durch Spritzgießen des Schaftmaterials gehalten ist.

10. Pleuelstange nach Anspruch 9, dadurch gekennzeichnet, daß das Schaftmaterial ein kurzfaserverstärkter Thermoplast ist.

11. Pleuelstange nach Anspruch 9 oder 10, dadurch gekennzeichnet, daß das erweiterte Schaftende (11) so ausgebildet ist, daß das durch Spritzgießen geformte Schaftmaterial im metallenen Pleuelkopf (15) verriegelt ist.

12. Pleuelstange nach Anspruch 8, dadurch gekennzeichnet, daß die beiden Hälften des zweiteiligen metallenen Pleuelkopfes (15) vor ihrer starren Zusammenspannung jeweils das erweiterte Schaftende (11) umgreifend angeordnet sind.

13. Pleuelstange nach Anspruch 12, dadurch gekennzeichnet, daß Mittel vorgesehen sind, die das erweiterte Schaftende (11) in Anlage an den Wandflächen der Ausnehmung (16) des metallenen Pleuelkopfes (15) pressen.

14. Pleuelstange nach Anspruch 13, dadurch gekennzeichnet, daß die Mittel zur Anpressung des erweiterten Schaftendes (11) in Anlage an den Wandflächen der Ausnehmung (16) des metallenen Pleuelkopfes (15) mindestens ein Beilageblech (17) und/oder in Gewindelöcher des metallenen Pleuelkopfes (15) eingeschraubte Schrauben (19) umfassen.

15. Pleuelstange nach einem der Ansprüche 1, 2, 6 oder 7, dadurch gekennzeichnet, daß der metallene Pleuelkopf (15) in Stellung um das erweiterte (14) Schaftende (11) herumgegossen ist.

16. Pleuelstange nach einem der Ansprüche 1 bis 15, dadurch gekennzeichnet, daß die Mittel zur Befestigung des Schaftes (10) am Lagerbügel (31) auch Stiftschrauben (30) umfassen, die am metallenen Pleuelkopf (15) befestigt und durch Durchgangslöcher (33) im Lagerbügel (31) hindurchgeschoben sind.

17. Pleuelstange nach einem der Ansprüche 1 bis 15, dadurch gekennzeichnet, daß die Mittel zur Befestigung des Schaftes am Lagerbügel (31) auch Schrauben umfassen, die durch im metallenen Pleuelkopf (15) und im Lagerbügel (31) angeordnete Durchgangslöcher hindurchgeschoben sind.

18. Pleuelstange nach einem der Ansprüche 1 bis 15, dadurch gekennzeichnet, daß die Mittel zur Befestigung des Schaftes (10) am Lagerbügel (31) auch Schrauben umfassen, die durch Durchgangslöcher (33) im Lagerbügel (31) hindurchgeschoben und in Gewindelöcher des metallenen Pleuelkopfes (15) eingeschraubt sind.

19. Getriebe zur Umsetzung von translatorischen Bewegungen in Drehbewegungen bzw. umgekehrt, dadurch gekennzeichnet, daß dasselbe eine Pleuelstange nach einem der Ansprüche 1 bis 18 aufweist.

**Revendications**

1. Bielle comportant un pied (12) et une tête et comprenant un corps (10) en matière plastique renforcée par des fibres, un chapeau (31) et un élément métallique (15), le chapeau (31) étant fixé sur le corps (10), caractérisé en ce que le corps (10) comprend une extrémité expansée localement (14) qui est maintenue prisonnière dans un évidement rentrant (16) dans l'élément métallique (15) pour fixer le corps (10) de façon rigide sur l'élément métallique (15).

2. Bielle selon la revendication 1, caractérisée en ce que l'élément métallique (15) présente la forme d'une selle.

3. Bielle selon la revendication 1 ou la revendication 2, caractérisée en ce que l'extrémité (11) du corps (10) est de section transversale rectangulaire et comporte deux côtés opposés, expansés pour former des saillies en forme de coin (15) qui viennent en appui sur les côtés d'un évidement rentrant (16) dans l'élément métallique (15).

4. Bielle selon la revendication 3, caractérisée en ce qu'elle comporte des moyens (17, 19) pour forcer les saillies en forme de coin (14) en contact de pression avec les extrémités de l'évidement (16).

5. Bielle selon la revendication 4, caractérisée en ce que les moyens destinés à forcer les saillies en forme de coin (14) en contact avec le côté de l'évidement (16) comprennent une cale d'épaisseur (17) et des vis (19) dans des alésages (20) dans l'élément métallique (15).

6. Bielle selon la revendication 2, caractérisée en ce que l'extrémité du corps (10) est de section transversale rectangulaire et l'évidement (16) présente la forme d'un tronc de pyramide.

7. Bielle selon la revendication 2, caractérisée en ce que l'extrémité du corps (10) est de section transversale circulaire et l'évidement (16) est de forme tronconique.

8. Bielle selon l'une quelconque des revendications 3, 6 ou 7, caractérisée en ce que l'élément métallique (15) est constitué de deux parties, chaque partie incorporant la moitié de l'évidement (16), les deux parties étant fixées de façon rigide entre elles.

9. Bielle selon l'une quelconque des revendications 3 à 8, caractérisée en ce que l'extrémité du corps (10) est fixée dans l'évidement (16) par son expansion par moulage par injection de la matière dans celui-ci.

10. Bielle selon la revendication 9, caractérisée en ce que la matière est une matière thermoplastique renforcée par des fibres courtes.

11. Bielle selon la revendication 9 ou la revendication 10, caractérisée en ce que l'extrémité (11) du corps (10) est adaptée de telle manière que la matière moulée par injection vient se verrouiller dans celle-ci.

12. Bielle selon la revendication 8, caractérisée en ce que les deux parties sont positionnées autour de l'extrémité (11) du corps (10) avant d'être réunies.

13. Bielle selon la revendication 12, caractérisée en ce qu'elle comporte des moyens pour forcer l'extrémité du corps (10) en contact sous pression avec les côtés de l'évidement (16).

14. Bielle selon la revendication 13, caractérisée en ce que les moyens destinés à forcer l'extrémité du corps (10) en contact sous pression avec les côtés de l'évidement (16) comprennent une cale d'épaisseur (17) et des vis (19) dans des alésages (20) de l'élément métallique (15).

15. Bielle selon l'une quelconque des revendications 1, 2, 6 ou 7, caractérisée en ce que l'élément metallique (15) est coulé en position autour de l'extrémité (11, 14) du corps (10).

16. Bielle selon l'une quelconque des revendications 1 à 15, caractérisée en ce que des moyens destinés à fixer le corps (10) sur le chapeau (31) comprennent également des goujons (30) qui sont fixés sur l'élément métallique (15) et qui traversent des trous (33) dans le chapeau (31).

17. Bielle selon l'une quelconque des revendications 1 à 15, caractérisée en ce que les moyens destinés à fixer le corps (10) sur le chapeau (31) comprennent également des boulons qui traversent des trous dans l'élément métallique (15) et dans le chapeau (31).

18. Bielle selon l'une quelconque des revendications 1 à 15, caractérisée en ce que les moyens destinés à fixer le corps (10) sur le chapeau (31) comprennent des boulons qui traversent des trous dans le chapeau (31) et des trous taraudés dans l'élément métallique (15).

19. Machine dans laquelle le mouvement alternatif est transformé en un mouvement rotatif ou vice versa comprenant une bielle selon l'une quelconque des revendications de 1 à 18.

# Fig.1

# Fig.2

# Fig.3